(19) Europäisches Patentamt / European Patent Office / Office européen des brevets

(11) **EP 2 976 999 B1**

(12) **EUROPEAN PATENT SPECIFICATION**

(45) Date of publication and mention
of the grant of the patent:
**03.08.2022 Bulletin 2022/31**

(21) Application number: **14771172.5**

(22) Date of filing: **28.02.2014**

(51) International Patent Classification (IPC):
**A61B 5/0245** *(2006.01)*    **A61B 5/022** *(2006.01)*
**A61B 5/02** *(2006.01)*    **A61B 5/00** *(2006.01)*

(52) Cooperative Patent Classification (CPC):
**A61B 5/02225; A61B 5/02007; A61B 5/7242;**
A61B 5/0245

(86) International application number:
**PCT/JP2014/001090**

(87) International publication number:
**WO 2014/147975 (25.09.2014 Gazette 2014/39)**

(54) **CIRCULATORY ORGAN FUNCTION ARITHMETIC CALCULATION DEVICE**

VORRICHTUNG ZUR ARITHMETISCHEN BERECHNUNG DER FUNKTION VON
KREISLAUFORGANEN

DISPOSITIF DE CALCUL ARITHMÉTIQUE DE FONCTION D'ORGANE CIRCULATOIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.03.2013 JP 2013055557**

(43) Date of publication of application:
**27.01.2016 Bulletin 2016/04**

(73) Proprietor: **Panasonic Intellectual Property
Management Co., Ltd.
Osaka-shi, Osaka 540-6207 (JP)**

(72) Inventors:
• **KUSAKABE, Tomoya
Osaka 540-6207 (JP)**

• **IDE, Kazuhiro
Osaka 540-6207 (JP)**

(74) Representative: **Grünecker Patent- und
Rechtsanwälte
PartG mbB
Leopoldstraße 4
80802 München (DE)**

(56) References cited:
EP-A1- 2 465 422    JP-A- 2004 195 204
JP-A- 2005 278 708    JP-A- 2005 279 248
JP-A- 2012 165 915    JP-A- 2013 009 940
JP-A- 2013 027 473    US-A1- 2014 194 755

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention relates to a circulatory organ function calculation device.

Patent document 1 describes a circulatory organ function calculation device including a constriction unit, a pressure detection unit, a pulse wave detection unit, and a characteristic quantity calculation unit. When the constriction unit constricts a measurement subject part of a measurement subject, the pressure detection unit detects constricted pressure generated by the constriction unit, and the pulse wave detection detects pulse wave information based on an output signal from the pressure detection unit. The characteristic quantity calculation unit calculates an index for the circulatory organ function of the measurement subject based on the pulse wave information. As the index for the circulatory organ function, the characteristic quantity calculation unit calculates the value of the upper base of a pulse wave amplitude pattern portion that conforms to a trapezoidal pattern.

EP 2 465 422 A1 relates to a circulatory function measurement device.

Patent Document 1: Japanese Laid-Open Patent Publication No. 2005-323853

The pulse wave information detected by the circulatory organ function calculation device of the above publication is affected by hardware characteristics, living body characteristics, and the measurement environment. The hardware characteristics may be the characteristics of, for example, the constriction unit and the pressure detection unit. The living body characteristics may be, for example, the age of the measurement subject, the size of the measurement subject part, the ratio of fat and muscle, sex, and race. The measurement environment may be, for example, the positional relationship of the measurement subject and the constriction unit and a change in the measurement position of the measurement subject. Thus, the circulatory organ function calculation device may output a calculation device deviated from the actual condition of the measurement subject.

Improvement in the calculation accuracy of the circulatory organ function calculation device would decrease the influence of hardware characteristics, living body characteristics, measurement environment, and the like on the calculation result.

The circulatory organ function device uses only the value of the upper base of a pulse wave amplitude pattern portion that conforms to a trapezoidal pattern and does not take into consideration other characteristics in the acquired pulse wave information. However, the other characteristics that are not taken into consideration may include those that reflect circulatory organ functions. Thus, there is room for improvement in the calculation accuracy of a circulatory organ function index.

It is an object of the present invention to provide a circulatory organ function calculation device that allows for improvement in the calculation accuracy of a circulatory organ function index.

The present invention relates to a circulatory organ function calculation device as defined in claim 1. Thus, the calculation accuracy of an index for a circulatory organ function can be improved.

In one example, the characteristic quantity calculation unit is configured to use, one of a varied amount of the cumulative addition ratios when the constriction pressure is varied by a predetermined amount, a gradient of a characteristic curve showing the relationship of the cumulative addition ratios and the constriction pressure when the constriction pressure is varied by a predetermined amount, an integral value of the characteristic curve of the cumulative addition ratios when the constriction pressure is varied by a predetermined amount.

In one example, the characteristic quantity calculation unit is configured to use one of a varied amount of the constriction pressure when the cumulative addition ratios are varied by a predetermined amount, a gradient of a characteristic curve showing the relationship of the constriction pressure and the cumulative addition ratios when the cumulative addition ratios are varied by a predetermined amount, an integral value of the characteristic curve of the constriction pressure when the cumulative addition ratios are varied by a predetermined amount.

In one example, the characteristic quantity of the circulatory organ function is a value quantitatively indicating a vascular condition, and the characteristic quantity calculation unit evaluates the vascular condition using a calculation result of the characteristic quantity.

In one example, the characteristic quantity calculation unit compares the characteristic quantity with a discriminant value used to distinguish a specific circulatory organ disease and generates a distinguishing result indicating a condition of the measurement subject related to the specific circulatory organ disease.

In one example, the multivariate analysis is a principal component analysis.

In one example, the characteristic quantity calculation unit specifies an envelope showing the relationship of the constriction pressure and the cumulative addition ratios, divides the envelope at predetermined varied amounts of the constriction pressures or predetermined varied amounts of the cumulative addition ratios, and uses values respectively corresponding to the divided portions of the envelope as the cumulative addition values.

In one example, the characteristic quantity calculation unit specifies a characteristic curve showing the relationship of the area of the pulse waves and constriction pressures when the pulse waves are detected, and substitutes differential values calculated by differentiating the characteristic curve to said equation.

The present invention provides a circulatory organ function calculation device that allows for improvement in the calculation accuracy of a circulatory organ function index.

Fig. 1 is a block diagram of a circulatory organ function calculation device.

Fig. 2 is a graph showing the relationship of constriction pressure detected by a pressure detection unit in the first embodiment with respect to time.

Fig. 3 is a graph showing the relationship of cuff pressure and pulse wave that are detected a pulse detection unit in the first embodiment.

Fig. 4A is a graph showing the relationship of the cuff pressure and pulse wave area, and Fig. 4B is a graph showing the relationship of the cuff pressure and a cumulatively added value of the pulse wave area.

Fig. 5 is a graph showing the relationship of the cuff pressure and a cumulative addition ratio of the pulse wave area.

Fig. 6 is a graph illustrating a plurality of variable quantities extracted from a characteristic line of the cumulative addition ratio of the pulse wave area.

Fig. 7 is a diagrammatic chart showing one example of an evaluation result in the first embodiment.

Fig. 8 is a graph illustrating a plurality of variable quantities extracted from a characteristic line of the cumulative addition ratio of the pulse wave area.

Fig. 9 is a diagrammatic chart showing one example of an evaluation result in a second embodiment.

Fig. 10 is a graph illustrating a plurality of variable quantities extracted from a characteristic line of the cumulative addition ratio of the pulse wave area.

Fig. 11 is a diagrammatic chart showing one example of an evaluation result in a third embodiment.

Fig. 12 is a graph showing a differential curve generated from the characteristic line of the pulse wave representative value.

Fig. 13 is a diagrammatic chart showing one example of an evaluation result in a fourth embodiment.

First Embodiment

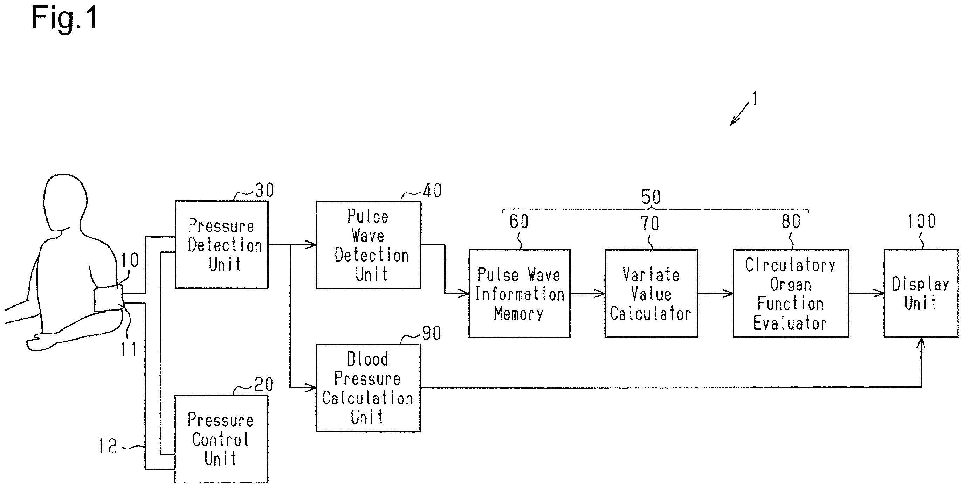

The configuration of a circulatory organ function calculation device 1 will now be described with reference to Fig. 1.

The circulatory organ function calculation device 1 includes a constriction unit 10, a pressure control unit 20, a pressure detection unit 30, a pulse wave detection unit 40, a characteristic quantity calculation unit 50, a blood pressure calculation unit 90, and a display unit 100.

The constriction unit 10 includes a cuff 11 and a tube 12. The cuff 11 is wound around a measurement subject part of a measurement subject. In a preferred example, the measurement subject part is the upper arm. The cuff 11 is inflated when supplied with air and constricts the measurement subject part. The tube 12 connects the cuff 11 to an air pump (not shown) and an air discharge valve (not shown) of the pressure control unit 20.

The pressure control unit 20 controls the supply and discharge of air to and from the constriction unit 10 to change the pressure of the cuff 11 (hereafter referred to as the constriction pressure P). The pressure control unit 20 controls the pressure of the cuff 11 with the air pump (not shown) and the air discharge valve (not shown). When increasing the constriction pressure P, the pressure control unit 20 drives the air pump and supplies air to the cuff 11. When decreasing the constriction pressure P, the pressure control unit 20 opens the air discharge valve and discharges air from the cuff 11.

The pressure detection unit 30 detects the constriction pressure P when the constriction unit 10 constricts the measurement subject part. The pressure detection unit 30 includes a pressure sensor (not shown) and an A/D converter (not shown). The pressure detection unit 30 converts the constriction pressure P of the constriction unit 10 detected by the pressure sensor to a pressure signal, which is formed by a digital signal, with the A/D converter. The pressure signal, which is converted by the pressure detection unit 30, is output to the pulse wave detection unit 40 and the blood pressure calculation unit 90.

The pulse wave detection unit 40 detects pulse wave information based on the pressure signal from the pressure detection unit 30. For example, the pulse wave detection unit 40, which includes a filter circuit, eliminates predetermined frequency components, such as direct current components, from the output signal of the pressure detection unit 30 at the filter circuit to generate a pulse wave signal. Then, the pulse wave detection unit 40 detects the amplitude of the pulse wave from the pulse wave signal and sends an amplitude signal indicating the detected amplitude of the pule wave to the characteristic quantity calculation unit 50.

The characteristic quantity calculation unit 50 includes a pulse wave information memory 60, a variate value calculator 70, and a circulatory organ function evaluator 80. The pulse wave information memory 60 stores the amplitude signal from the pulse wave detection unit 40 in association with the constriction pressure P. The variate value calculator 70 calculates or extracts variate values from the amplitude of the pulse wave stored in the pulse wave information memory 60. The circulatory organ function evaluator 80 substitutes the variate values to a preset calculation equation to evaluate the circulatory organ function of the measurement subject. The circulatory organ function evaluator 80 sends the evaluation result for the circulatory organ function of the measurement subject to the display unit 100.

The blood pressure calculation unit 90 estimates or calculates the systolic blood pressure, the diastolic blood pressure, and the average blood pressure from the relationship of the constriction pressure P detected by the pressure detection unit 30 and the amplitude of the pulse wave detected by the pulse wave detection unit 40 using predetermined algorithms of the oscillometric method or the like. The blood pressure calculation unit 90 sends the calculation result of the blood pressure to the display unit 100.

The display unit 100 includes a crystal liquid screen. The display unit 100 shows the evaluation result of the circulatory organ function evaluator 80 and the systolic, diastolic, and average blood pressure of the measurement subject calculated by the blood pressure calculation unit 90.

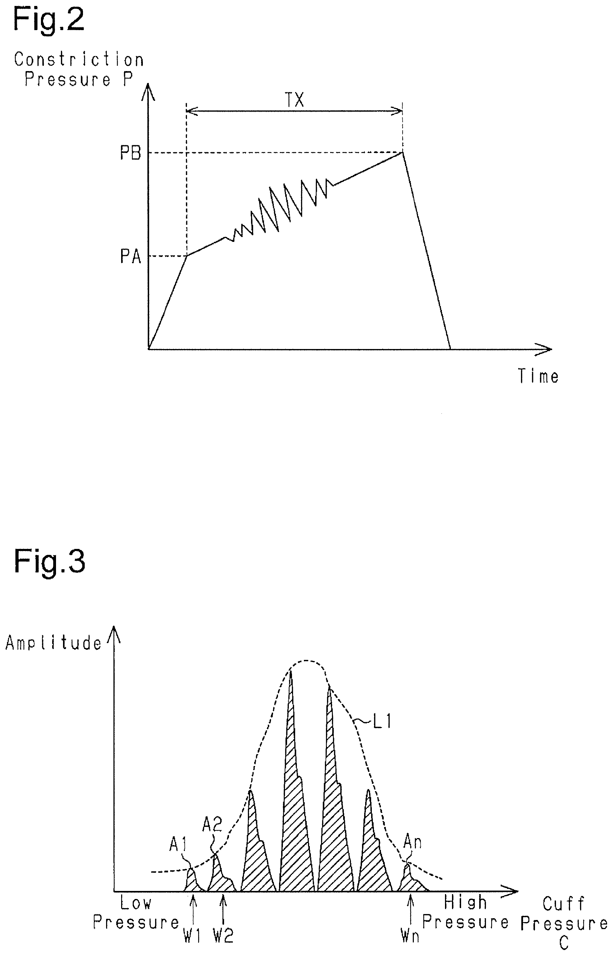

The evaluation of the circulatory organ function will now be described with reference to Figs. 2 to 6.

As shown in Fig. 2, when evaluating the circulatory organ function, the pressure control unit 20 varies the constriction pressure P. For example, the pressure control unit 20 inflates the cuff 11 and raises the constriction pressure P to a first pressure PA. After raising the constriction pressure P to the first pressure PA, the pressure control unit 20 decreases the rising speed of the constriction pressure P. The pressure control unit 20 raises the constriction pressure P to a second pressure PB, which is higher than the first pressure PA. Then, the pressure control unit 20 discharges air from the cuff 11 to lower the constriction pressure P.

The first pressure Pa and the second pressure PB are set to allow for measurement of a typical blood pressure range. During a period in which the pressure control unit 20 raises the constriction pressure P from the first pressure PA to the second pressure PB (hereafter referred to as the pulse wave detection period TX), the constriction pressure P detected by the pressure detection unit 30 varies for each pulse wave W that corresponds to a single cardiac cycle.

In the example of Fig. 3, pulse waves W1, W2, ..., Wn are shown in order measured when the cuff pressure C was raised. The horizontal axis of Fig. 3 shows the pressure of the constriction unit 10 corresponding to the amount of air supplied to the constriction unit 10 during the pulse wave detection period TX (hereafter referred to as the cuff pressure C). The steep change in the constriction pressure P that occurs when the cuff pressure C is changing at a controlled speed indicates the generation of a pulse wave W. The cuff pressure C may be a value obtained by eliminating the influence of the pulse wave W from the constriction pressure P. The pulse wave signal detected by the pulse wave detection unit 40 corresponds to a change in the constriction pressure P of the cuff pressure C.

The pulse waves W1, W2, ..., Wn reflect changes in the blood pressure volume that corresponds to the pressure difference between the outer and inner sides of the block vessel. Thus, it is understood that this allows complex information including the blood pressure, the circulatory organ function, and the dynamic function of the living body to be obtained from the pulse waves W1, W2, ..., Wn. The characteristic quantity calculation unit 50 extracts optimal information corresponding to the desired circulatory organ function from the pulse waves W1, W2, ..., Wn to calculate the characteristic quality of the circulatory organ function.

For example, from the information stored in the pulse wave information memory 60, the variate value calculator 70 calculates or extracts information indicating the relationship of the area of each pulse wave (hereafter referred to as the pulse wave area A) and the cuff pressure C (e.g., data pairs (C1, A1) to (Cn, An)). Then, the calculated or extracted information is sent to the circulatory organ function evaluator 80 as a variate value. The pulse wave area A may be calculated by adding pulse wave signals during a period in which a single pulse wave W is detected. When an n number of pulse waves W are detected during a pulse wave detection period TX, the pulse area A of the pulse wave W1 that is the first one to be detected to the pulse area An of the pulse wave W1 that is the nth one to be detected are each calculated. Each pulse wave area A is one example of a representative value of each pulse wave W.

Envelope L1 is obtained from the relationship of the cuff pressure and the pulse area A. The pulse area A shows a change that is characteristic to the measurement subject when finely increasing the pressure of the constriction unit 10. Thus, the envelope L1 is ridge-shaped in a manner that is characteristic to the measurement subject. The shape of the envelope L1 differs between circulatory organ diseases. The envelope L1 may be an envelope of the amplitudes of a plurality of pulse waves W or the envelope of the areas of a plurality of pulse waves W.

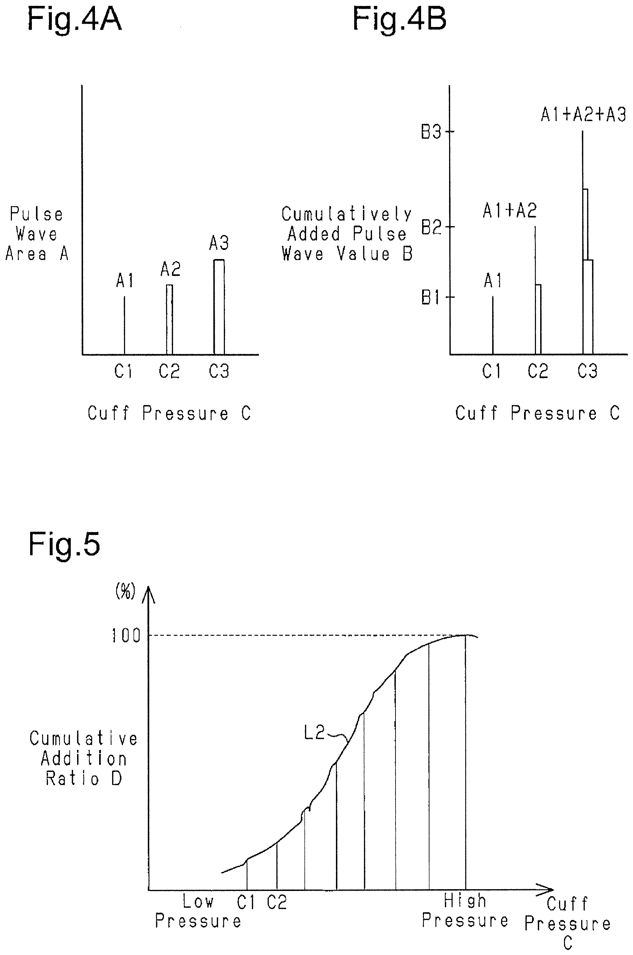

The circulatory organ function evaluator 80 uses the output from the variate value calculator 70 as data pairs obtained by combining the cuff pressure C, which indicates the detected order of the pulse waves W1 to Wn, and the pulse wave area A. The circulatory organ function evaluator 80 performs a calculation using the pulse area A and a predetermined function to generate converted information. For example, the circulatory organ function evaluator 80 calculates a cumulatively added pulse wave value B such as the cumulatively added values of the pulse wave areas shown in Fig. 4B from the pulse wave areas A shown in Fig. 4A. The cumulatively added pulse wave value B is one example of converted information. The cumulatively added pulse wave value B1 corresponding to cuff pressure C1 is pulse area A1. The cumulatively added pulse wave value B2 corresponding to cuff pressure C2 is pulse wave area A1+A2. The cumulatively added pulse wave value B3 corresponding to cuff pressure C3 is pulse wave area A1+A2+A3. The circulatory organ function evaluator 80 calculates the variate value using a plurality of coordinates (C1, B1), (C2, B2), ..., (Cn, Bn), which are generated by combining the cuff pressure (Cn) and the cumulatively added pulse wave value (Bn). The coordinates may be referred to as data pairs that combine the cuff pressure C, which indicates the pulse waves W1 to Wn in detected order, and the cumulatively added pulse wave value B. In this manner, the cuff pressure C during the pulse wave detection period TX functions as a variable so that data pairs related with the pulse wave can be obtained.

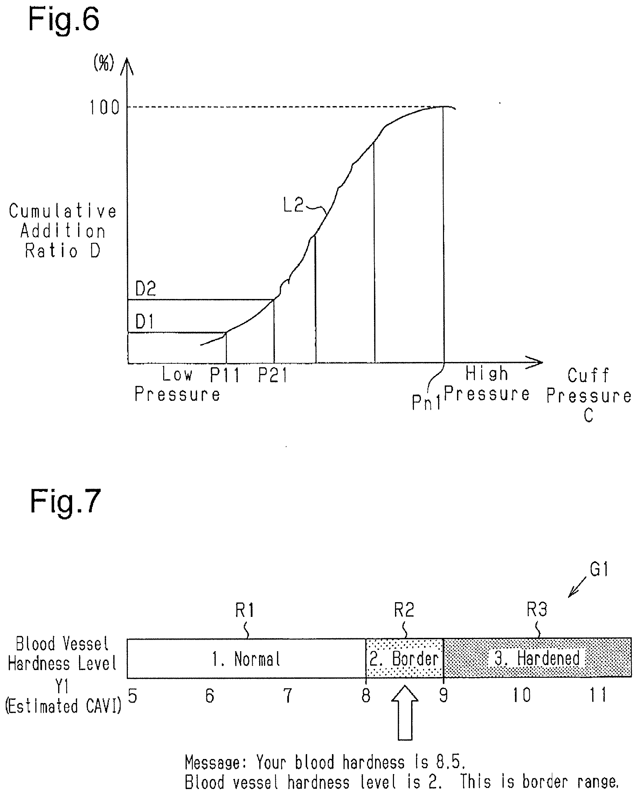

In Figs. 5 and 6, the horizontal axes represent the cuff pressure C, and the vertical axes represent the cumulative addition ratio D. The cumulative addition ratio D, which is also referred to as a normalized cumulatively added pulse wave value, is a value (%) normalizing each of the cumulatively added pulse wave values B1 to Bn relative to the maximum cumulatively added pulse wave value Bn (100%), which is obtained by cumulatively adding the areas A1 to An of every one of the pulse waves W1 to Wn detected during the pulse wave detection period TX. The circulatory organ function evaluator 80 sequentially adds the detected pulse wave areas A from a lower cuff pressure C to calculate the cumulative addition ratio D. A characteristic curve, or envelope L2, indicating the relationship of the cuff pressure C and the cumulative addition ratio D reflects changes in the vascular volume and represents the vascular dynamic characteristics. As the constriction pressure of the cuff 11 increases, the envelope L2 of the cumulative addition ratio D first slowly rises and then suddenly rises at a characteristic cuff pressure. After exceeding the characteristic cuff pressure, the rising rate of the envelope L2 gradually decreases. The characteristic cuff pressure may be referred to as the cuff pressure when the increase in the cumulative addition ratio D is maximal.

In the horizontal axis of Fig. 6, pressures P11, P21, ..., Pn1 sequentially indicate the cuff pressure at fixed intervals from a lower pressure. A data row, which is formed by data pairs (P11, D1), (P21, D2), ..., (Pn1, Dn) that combine the cuff pressure C in order from a lower pressure with the cumulative addition ratio D, may be extracted from a characteristic curve, or the envelope L2, which indicates the relationship of the pressures P11, P21, ..., Pn1 and the cumulative addition ratio D.

The circulatory organ function evaluator 80 includes equation (1) used to calculate a characteristic quantity Y1. The circulatory organ function evaluator 80 substitutes the variate values (D1 ... Dn) to equation (1) to calculate the characteristic quantity Y.

$$Y1 = b0 + b1 \times D1 + b2 \times D2 + ... + bn \times Dn \quad ...(1)$$

The characteristic quantity Y1 quantitatively indicates the blood vessel hardening level, which serves as an index of the vascular condition.

The coefficients b0, b1, ..., bn of equation (1) for calculating the characteristic quantity Y1 may be a PLS model coefficient obtained through a PLS analysis using the cardio-ankle vascular index (CAVI), which is a typical blood vessel hardness index, as an objective variable.

To determine each coefficient, the pulse waves of test subjects having different CAVI values were measured. For example, the constriction unit 10, the pressure control unit 20, the pressure detection unit 30, and the pulse wave detection unit 40 may be used to obtain the data row of "(P11, D1), (P21, D2), ..., (Pn1, Dn)" from each test subject. The coefficients of equation (1) may be determined by performing the PLS analysis on the data rows of the test subjects.

As shown in Fig. 7, the circulatory organ function evaluator 80 includes a gauge G1 for the characteristic quantity Y1. The circulatory organ function evaluator 80 shows the calculated characteristic quantity Y1 on the gauge G1 as the blood vessel hardness or blood vessel hardness level indicating the circulatory organ function of the measurement subject. In the illustrated example, the gauge G1 includes ranges R1, R2, and R3. Underneath the ranges R1, R2, and R3, corresponding values of the characteristic quantity Y1 are shown. Range R1 of the gauge G1 indicates that the blood vessel hardness is sufficiently lower than a disease risk border value. Range R2 of the gauge G1 indicates that the blood vessel hardness is in the vicinity of the disease risk border value. Range R3 of the gauge G1 indicates that the blood vessel hardness is significantly higher than a disease risk border value. The evaluation result may be shown on the display unit 100 as a message of "Your blood vessel hardness is 8.5. The blood vessel hardness level is 2. This is the border range."

The operation of the circulatory organ function calculation device 1 will now be described.

The circulatory organ function calculation device 1 stores an equation used for multivariate analysis in advance. Variable quantities calculated or extracted from the pulse wave information are substituted to the variables of the multivariate analysis equation, which is stored in advance, to calculate the characteristic quantity Y1, which is an index for a circulatory organ function. The calculation of the characteristic quantity Y1 uses the variate values calculated or extracted from the pulse wave information. This allows the circulatory organ function index to be calculated with high accuracy. In a hypothetical example that calculates an index of a circulatory organ function using a single variate obtained from the pulse wave information, the calculation accuracy is low.

The circulatory organ function calculation device 1 has the advantages described below.

(1) The characteristic quantity calculation unit 50 evaluates the circulatory organ function by substituting the variable quantities calculated or extracted from the pulse wave information to the variables of equation (1) having variables and coefficients obtained in advance through a multivariate analysis. This improves the calculation accuracy of the characteristic quantity Y1, which serves as an index for a circulatory organ function.

(2) The characteristic quantity calculation unit 50 evaluates the circulatory organ function using the cumulative addition ratio D (envelope L2), which serves as converted information obtained from the envelope L1 through a function calculation. This allows the characteristic quantity Y1 to be obtained that represents the circulatory organ function characteristics unique to the measurement subject. Further, the measurement sensitivity is increased.

(3) The characteristic quantity calculation unit 50 uses a value converted from pulse areas A of the pulse waves W as a variate value to calculate the characteristic quantity of the circulatory organ function. Thus, the calculation result of the characteristic quantity of the circulatory organ function has stronger resistance to data noise than when using the peak height of the pulse wave W as a variate. This improves the calculation accuracy of the characteristic quantity Y1 for a circulatory organ function.

(4) The characteristic quantity calculation unit 50 obtains discrete data of pulse waves representing a circulatory organ function as continuous data to calculate a variate value with the envelope L1. Thus, variables that are not dependent on the heart rate may be obtained, and the calculation accuracy of the circulatory organ function is increased.

(5) The pressure of the cuff 11 is controlled to be finely increased during the pulse wave detection period TX. This configuration allows for the measurement of the pulse wave under a vascular condition before the cuff 11 constricts the measurement subject part and closes the blood vessel and the measurement of the pulse wave under a vascular condition when the blood vessel is closed. Further, this configuration allows for the detection of the pulse wave under a vascular condition that is more natural than a comparative example that detects the pulse wave W while decreasing the pressure.

(6) The characteristic quantity calculation unit 50 uses every one of the pulse waves W detected during the pulse wave detection period TX to calculate the variate. Thus, the calculation accuracy of the circulatory organ function is higher than a comparative example that does not use all of the pulse waves W detected during the pulse wave detection period TX.

(7) The display unit 100 includes a liquid crystal screen. The display unit 100 allows for prompt indication of the evaluation result of the circulatory organ function directly obtained from the characteristic quantity calculation unit 50.

(8) The circulatory organ function calculation device 1 is configured to calculate a characteristic quantity that quantitatively indicates a vascular condition or a circulatory organ function. The characteristic quantity may be shown on the gauge G1 with a numerical value indicating a vascular condition or a circulatory organ function. This allows the measurement subject to easily acknowledge the level of the circulatory organ function.

Second Embodiment

The circulatory organ function calculation device 1 of the second embodiment differs from the first embodiment in that discriminant analysis is performed as the multivariate analysis.

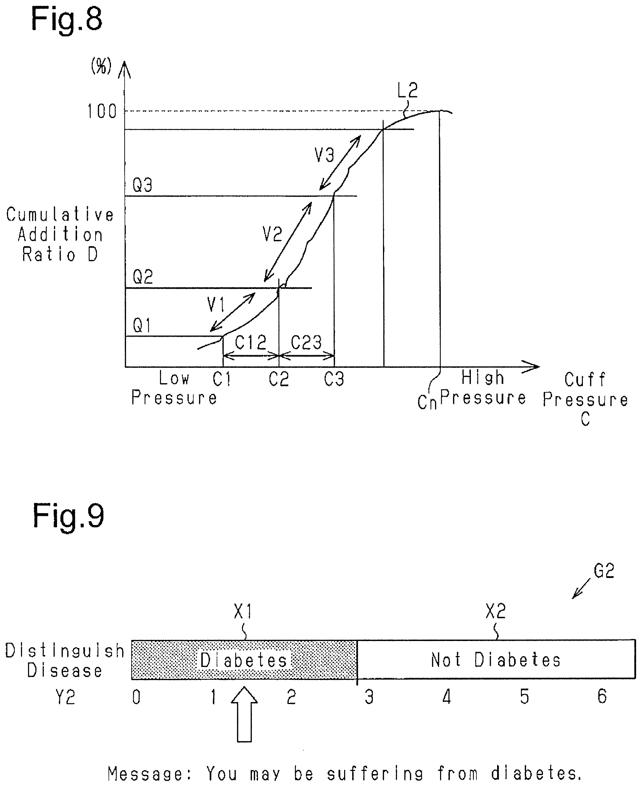

Referring to Fig. 8, the characteristic quantity calculation unit 50 extracts gradients V from the envelope L2 of the cumulative addition ratio D and uses the gradients V as variate values. Each gradient V is the varied amount of the cumulative addition ratio D relative to a predetermined cuff pressure varied amount during the pulse wave detection period TX.

For example, gradient V1 corresponding to a cuff pressure varied amount C12 is calculated from "$V1=(Q2-Q1)/(C2-C1)$". Gradient V2 corresponding to a cuff pressure varied amount C23 is calculated from "$V2=(Q3-Q2)/(C3-C2)$". The cuff pressures C1, C2, ..., Cn correspond to pulse waves W1 to Wn in order of detection. Here, "$Cn-C(n-1)$" indicates a predetermined cuff pressure varied amount, and "Qn" represents the cumulative addition ratio D corresponding to "Cn". The characteristic quantity calculation unit 50 generates the data pairs of "(C1,V1), (C2,V2), ..., (Cn,Vn)" in order from a lower pressure using the envelope L2 of the cumulatively addition ratio D.

The circulatory organ function evaluator 80 includes equation (2) used to calculate a characteristic quantity Y2. The circulatory organ function evaluator 80 calculates the characteristic quantity Y2 by substituting the variate value calculated by the variate value calculator 70 to equation (2).

$$Y2=c1\times V1+c2\times V2+\ldots+cn\times Vn \quad ...(2)$$

The characteristic quantity Y2 represents a value corresponding to a discriminant value for diabetes as a vascular condition index.

The coefficients c1, c2, ..., cn that are included in the equation (2) used to calculate the characteristic quantity Y2 may be, for example, a coefficient of a linear discriminant function expression obtained through a discriminant analysis that uses the existence of diabetes, which is a disease related a circulatory organ function, as an objective variable.

To determine each coefficient, the pulse waves may be measured in test subjects who are suffering from diabetes and test subjects who are not suffering from diabetes. For example, the constriction unit 10, the pressure control unit 20, the pressure detection unit 30, and the pulse wave detection unit 40 of the circulatory organ function calculation device 1 are used to obtain the data row of (C1,V1), (C2,V2), ..., (Cn,Vn) from each of the measurement subjects. The discriminant analysis may be conducted on the data rows of the test subjects to determine the coefficients of equation (2).

As shown in Fig. 9, the circulatory organ function evaluator 80 includes a gauge G2 for the characteristic quantity Y2. The circulatory organ function evaluator 80 shows the calculated characteristic quantity Y2 on the gauge G2 as the possibility the measurement subject has diabetes, which is related to a circulatory organ function of the measurement subject. In the illustrated example, the gauge G2 includes ranges X1 and X2. Underneath the ranges X1 and X2, corresponding values of the characteristic quantity Y2 are shown. Range X2 of the gauge G2 indicates that the possibility of diabetes is low. Range X1 indicates that there is a high possibility of diabetes. The evaluation result may be shown on the display unit 100 as a message of "You may be suffering from diabetes."

In addition to advantages (1) to (7) of the first embodiment, the circulatory organ function calculation device 1 of the second embodiment has the following advantage.

(9) The circulatory organ function evaluator 80 performs the discriminant analysis using the varied amount of the cumulative addition ratio D for each predetermined cuff pressure varied amount or the gradient V of the envelope L2 of the cumulative addition ratio D. For example, gradients V may be obtained from the envelope L2, and pressures corresponding to the gradients V may be obtained. The variate values may be calculated or extracted from the pulse wave information without depending on the number of pulse waves W detected during the pulse wave detection period TX or the heart rate. This allows for a reduction in measurement errors caused by the heart rate, which may differ for each measurement.

Third Embodiment

The circulatory organ function calculation device 1 of the third embodiment differs from the first embodiment in that the circulatory organ function calculation device 1 is configured to perform a principal component analysis as the multivariate analysis.

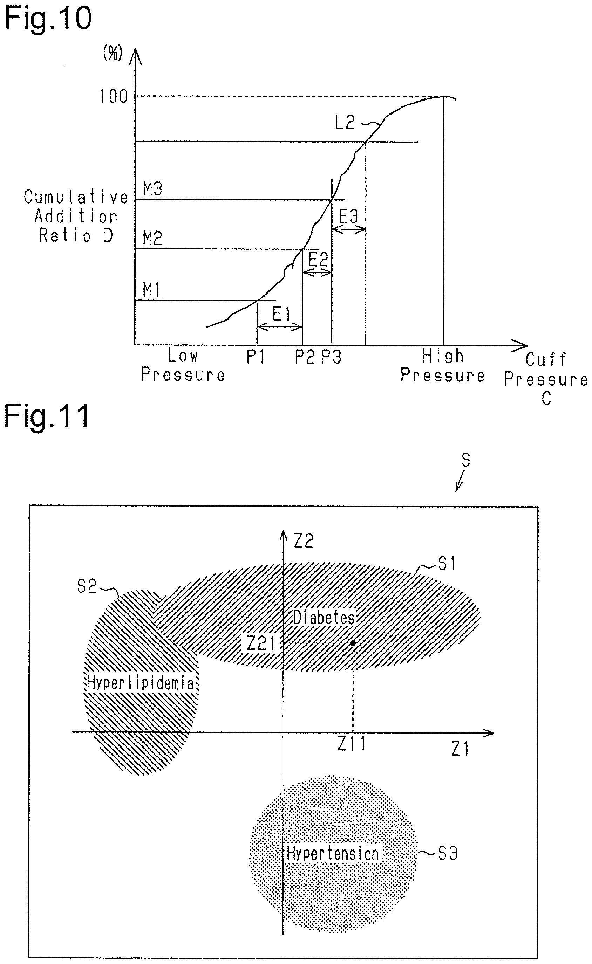

Referring to Fig. 10, the characteristic quantity calculation unit 50 calculates cuff pressure varied amounts (hereafter referred to as "the pressure differences E") respectively corresponding to predetermined varied amounts of the cumulative addition ratio D from the envelope L2 of the cumulative addition ratio D, and uses the pressure differences E as variate values.

In the illustrated example, pressure difference E1 is calculated as "E1=P2-P1". The characteristic quantity calculation unit 50 generates the data pairs of "(M1,E1), (M2,E2), ..., (Mn,En)" in order from a lower pressure using the envelope L2 of the cumulative addition ratio D. Here, "M1,M2, ..., Mn" may be cumulative addition ratios D respective corresponding to pressures P1, P2, ..., Pn. Further, "Mn-M(n-1)" represents the varied amount of the cumulative addition ratio D corresponding to the pulse wave that is the nth one to be detected.

The circulatory organ function evaluator 80 includes equations (3) and (4) used to calculate characteristic quantities Z11 and Z21. The circulatory organ function evaluator 80 substitutes the variate values calculated by the variate value calculator 70 to equations (3) and (4) to calculate the characteristic quantities Z11 and Z21.

$$Z11 = a11 \times E1 + a12 \times E2 + \ldots + a1n \times En \quad \ldots(3)$$

$$Z21 = a21 \times E1 + a22 \times E2 + \ldots + a2n \times En \quad \ldots(4)$$

The characteristic quantities Z11 and Z21 are indexes for different first and second vascular conditions.

Coefficients a11, a12, ..., a1n that are included in equation (3), which is used to calculate the characteristic quantity Z11, and coefficients a21, a22, ..., a2n that are included in equation (4), which is used to calculate the characteristic quantity Z21, may be determined in advance through a principal component analysis serving as a multivariate analysis.

Referring to Fig. 11, the circulatory organ function evaluator 80 holds a graph S including an axis for characteristic quantity Z1 and an axis for characteristic quantity Z2. The circulatory organ function evaluator 80 plots points including the characteristic quantity Z11 and the characteristic quantity Z21, which are calculated with the equations (3) and (4), to evaluate the circulatory organ function of the measurement subject.

The relationship of the characteristic quantities Z1 and Z2 and the circulatory organ function is determined by giving a meaning to the characteristic quantities Z1 and Z2 through the principal component analysis. For example, in Fig. 11, the range S2 corresponding to the small characteristic quantity Z1 indicates that the measurement subject is suffering from or may suffer from hyperlipidemia. Range S3 corresponding to the small characteristic quantity Z2 indicates that the measurement subject is suffering from or may suffer from hypertension.

The principal component analysis used to determine each coefficient of the equations (3) and (4) will now be described.

To determine each coefficient, the pulse waves may be measured for test subjects having different vascular conditions. For example, the data row of "(M1,E1), (M2,E2), ..., (Mn,En)" is obtained from each of the test subjects using the constriction unit 10, the pressure control unit 20, the pressure detection unit 30, and the pulse wave detection unit 40 of the circulatory organ function calculation device 1. The principal component analysis is performed on the data rows of the test subjects to determine the coefficients.

First, the data row of "(M1,E1), (M2,E2), ..., (Mn,En)" is used to obtain an equation for calculating a first principal component and second to nth principal components. Then, the data rows of the test subjects are substituted to an equation for calculating each principal component, and a score of each principal component is calculated for each test subject.

Then, a scatter diagram is formed using the score of each principal component for each test subject. The scatter diagram is used to add a meaning to each principal component or classify the principal components.

In addition to advantages (1) to (8) of the first embodiment, the circulatory organ function calculation device 1 of the third embodiment has the following advantages.

(10) The circulatory organ function calculation device 1 is configured to use multivariate equations obtained through a principal component analysis, which is a multivariate analysis. This allows for a reduction in variations of a multivariate and a robust characteristic quantity may be calculated.

(11) The characteristic quantity calculation unit 50 calculates the characteristic quantities Z11 and Z21 from the pulse waves and evaluates a circulatory organ function with the graph S that has axes for the two calculated characteristic quantities Z11 and Z21. The two calculated characteristic quantities Z11 and Z21 are simultaneously shown with ranges related to circulatory organ functions in the graph S. Thus, the measurement subject may easily understand the results.

Fourth Embodiment

The circulatory organ function calculation device 1 of the fourth embodiment differs in that a qualitative analysis is performed as the multivariate analysis.

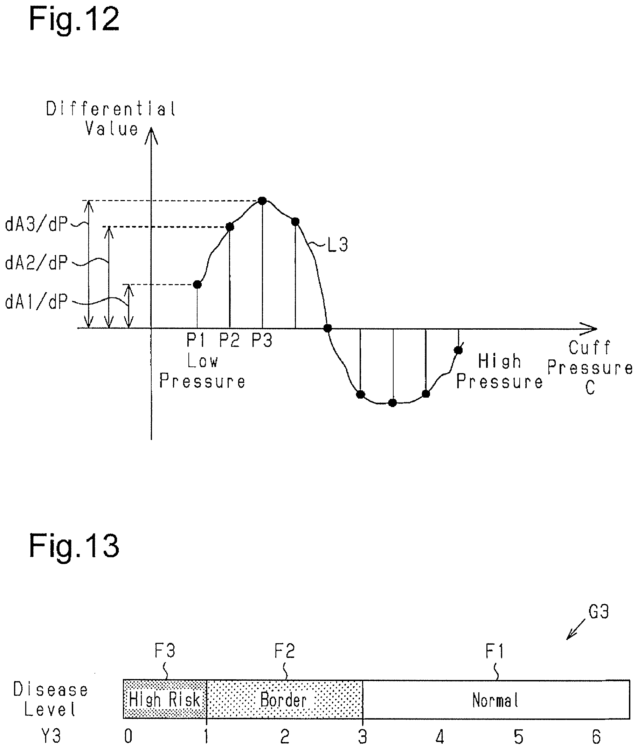

Fig. 12 shows a differential curve L3 generated by differentiating a characteristic curve (e.g., the envelope L1 of Fig. 3), which shows the characteristics of the cuff pressure C and a representative value of a pulse wave such as the area (or amplitude) of a pulse wave W. The horizontal axis of Fig. 12 shows the cuff pressure C. The vertical axis of Fig. 12 shows a pulse wave area envelope differential value as converted information. The characteristic curve (e.g., the envelope L1), which shows the characteristics of the cuff pressure C and the area (or amplitude) of a pulse wave W, is typically ridge-shaped. Thus, the differential curve L3 of the characteristic curve has a laterally lying S-shaped form. The differential curve L3 shows the varied amount of the area (or amplitude) of the pulse wave W relative to a change in the cuff pressure. When the cuff pressure C changes at a fixed speed, the differential curve L3 shows the varying sped of the area (or amplitude) of the pulse wave W. The differential curve L3 shows the vascular dynamic characteristics.

Pressures P1, P2, ..., Pn of the horizontal axis in Fig. 12 indicate predetermined cuff pressures C. The characteristic quantity calculation unit 50 extracts, from the differential curve L3, the data pairs of "(P1,dA1/dP), (P2,dA2/dP), ..., (Pn,dAn/dP)" in order from a lower pressure. The differential value "dAn/dP" included in each data pair may be an approximation value calculated in accordance with equation (5).

$$dA_n / dP = (A_{n+1} - A_n)/(P_{n+1} - P_n) \qquad \cdots (5)$$

The circulatory organ function evaluator 80 includes equation (6) to calculate the characteristic quantity Y3. The circulatory organ function evaluator 80 substitutes the variate values calculated by the variate value calculator 70 to equation (6) to calculate the characteristic quantity Y3.

$$Y3 = \sqrt{(R11 - R12)^2 + (R21 - R22)^2 + \cdots + (Rn1 - Rn2)^2} \qquad \cdots (6)$$

In equation (6), "Rn" is an abbreviated notation for "dAn/dP". The equation (6) may be a discriminant equation based on the Euclidean distance using an objective variable as a group of diseases. The circulatory organ function evaluator 80 qualitatively evaluates the measurement result in accordance with a discriminant vale indicating the risk level of a disease. The terms R11, R21, ..., Rn1 in equation (6) are reference values that are determined in advance, and "R12, R22, ..., Rn2" are calculated values corresponding to the measurement results of the measurement subject.

As shown in Fig. 13, the circulatory organ function evaluator 80 includes a gauge G3 for the characteristic quantity Y3. The circulatory organ function evaluator 80 shows the calculated characteristic quantity Y3 on the gauge G3 to indicate the risk of disease related with a circulatory organ function of the measurement subject. In the illustrated example, the gauge G3 includes ranges F1, F2, and F3. Underneath the ranges F1, F2, and F3, corresponding values of the characteristic quantity Y3 are shown. Range F1 of the gauge G3 indicates that the risk of disease is low. Range F3 of the gauge G3 indicates that the risk of disease is high. Range F2 of the gauge G3 indicates that the risk of disease is intermediate. The fourth embodiment has advantages (1) to (6) of the first embodiment.

The above embodiments may be modified as described below. Some of the modified examples may be combined with one another.

In the third embodiment, instead of performing the principal component analysis to calculate the characteristic quantities Z11 and Z21, the characteristic quantity calculation unit 50 may perform multiple regression analysis to calculate the characteristic quantities Z11 and Z21. In this case, the equation that is used is obtained in advance through a multiple regression analysis. This configuration allows a regression model that is optimal for the objective variable to be formed from explanatory variables and allows accurate calculation values to be obtained. Instead of a multiple regression analysis, canonical correlation analysis may be performed. This allows for the calculation of a coefficient of which the correlation is maximal relative to multiple circulatory organ functions. Further, the characteristic quantity of each circulatory organ function may be accurately calculated.

In the third embodiment, instead of or in addition to showing the evaluation result of the circulatory organ function with the graph S, the evaluation result of the circulatory organ function may be indicated with a text message or be notified with a voice message. An example of a test message is "high risk range of arteriosclerosis". In addition to the ranges shown on the graph S, circulatory organ function levels may be shown in correspondence to characteristic quantities Z11 and Z21, such as "hardness of blood vessels is level 4" and "clogging is level 6".

Instead of using the two characteristic quantities Z11 and Z21, the characteristic quantity calculation unit 50 of the third embodiment may calculate one characteristic quantity or three or more characteristic quantities. When calculating three or more characteristic quantities, a circulatory organ function may be evaluated with further detailed numerical values or classifications.

The pressure of the cuff 11 during the pulse wave detection period TX does not have to be controlled to increase at a fine speed. For example, the pressure of the cuff 11 during the pulse wave detection period TX may be controlled to decrease at a fine speed. When decreased at a fine speed, the pressure control unit 20 first inflates the cuff 11 and raises the constriction pressure P to the second pressure PB. Then, the constriction pressure is gradually lowered from the second pressure PB to the first pressure PA. Subsequently, the pressure control unit 20 discharges air from the cuff 11. In the pulse wave measurement when finely decreasing the pressure, the generation points of pulse wave appear more clearly than when finely increasing the pressure. Thus, the blood pressure may be accurately measured.

The circulatory organ function calculation device 1 in each of the above embodiments does not have to include the display unit 100. Instead of or in addition to the display unit 100, a connection terminal may be added to send the evaluation result to an external device. In this case, the evaluation result may be shown on the external device. Further, various applications may be easily prepared in an external device. This improves the convenience for storing and analyzing data.

It is preferred that the characteristic quantity calculation unit 50 of each of the above embodiments calculate the characteristic quantities (Y1, Y2, Y3, Z11, and Z21) using all of the pulse waves W detected during the pulse wave detection period TX. However, the characteristic quantity calculation unit 50 may calculate the characteristic quantities (Y1, Y2, Y3, Z11, and Z21) using only some, preferably, two or more, of the pulse waves W detected during the pulse wave detection period TX.

Instead of using the cuff pressure C, the characteristic quantity calculation unit 50 may use the pressure difference between the outer and inner side of the blood vessels. The pressure difference between the outer and inner side of the blood vessels may be calculated from the difference between the average blood pressure and the constriction pressure.

The measurement subject part of the measurement subject is not limited to the upper arm and may be, for example, the wrist or the lower leg.

Fig. 4B is a preferred example of a map that directly shows the relationship of constriction pressures and cumulatively added pulse wave values. Figs. 5, 6, 8, 10, and 12 are maps that indirectly show the relationship of constriction pressures and cumulatively added pulse wave values.

The subject matter of the present invention may be in fewer features than all of the features specified in the disclosed embodiments. Thus, the claims are incorporated in the detailed description and are each asserted as an individual embodiment. The present invention includes the scope and equivalence of the appended claims.

**Claims**

1. A circulatory organ function calculation device comprising:

   a constriction unit (10) configured to constrict a measurement subject part of a measurement subject;
   a pressure detection unit (30) that detects constriction pressure generated by the constriction unit;
   a pressure control unit (20) that varies the constriction pressure;
   a pulse wave detection unit (40) that detects pulse waves based on an output signal from the pressure detection unit during a constriction pressure varying process performed by the pressure control unit; and
   **characterized by**
   a characteristic quantity calculation unit (50) that:
   generates cumulatively added pulse wave values by cumulatively adding an area of the pulse waves which respectively correspond to constricting pressures, calculates cumulative addition ratios by normalizing the cumulatively added pulse wave values, and substitutes the cumulative addition ratios into an equation including variables and coefficients that are obtained in advance through a multivariate analysis.

2. The circulatory organ calculation device according to claim 1, wherein the characteristic quantity calculation unit (50) is configured to use one of a varied amount of the cumulative addition ratios when the constriction pressure is varied by a predetermined amount,

   a gradient of a characteristic curve showing the relationship of the cumulative addition ratios and the constriction pressure when the constriction pressure is varied by a predetermined amount,
   an integral value of the characteristic curve of the cumulative addition ratios when the constriction pressure is varied by a predetermined amount.

3. The circulatory organ calculation device according to claim 1, wherein the characteristic quantity calculation unit (50) is configured to use one of

   a varied amount of the constriction pressure when the cumulative addition ratios are varied by a predetermined amount,
   a gradient of a characteristic curve showing the relationship of the constriction pressure and the cumulative addition ratios when the cumulative addition ratios are varied by a predetermined amount,
   an integral value of the characteristic curve of the constriction pressure when the cumulative addition ratios are varied by a predetermined amount.

4. The circulatory organ function calculation device according to any one of claims 1 to 3, wherein

   the characteristic quantity of the circulatory organ function is a value quantitatively indicating a vascular condition, and
   the characteristic quantity calculation unit (50) evaluates the vascular condition using a calculation result of the characteristic quantity.

5. The circulatory organ function calculation device according to any one of claims 1 to 3, wherein the characteristic quantity calculation unit (50) compares the characteristic quantity with a discriminant value used to distinguish a specific circulatory organ disease and generates a distinguishing result indicating a condition of the measurement subject related to the specific circulatory organ disease.

6. The circulatory organ function calculation device according to any one of claims 1 to 4, wherein the multivariate analysis is a principal component analysis.

7. The circulatory organ function calculation device according to one of claims 1 to 6, wherein the characteristic quantity calculation unit (50)
   specifies an envelope showing the relationship of the constriction pressure and the cumulative addition ratios, divides the envelope at predetermined varied amounts of the constriction pressures or predetermined varied amounts of the cumulative addition ratios, and uses values respectively corresponding to the divided portions of the envelope as the cumulative addition ratios.

8. The circulatory organ function calculation device according to claim 1, wherein the characteristic quantity calculation unit (50) specifies a characteristic curve showing the relationship of the area of the pulse waves and constriction pressures when the pulse waves are detected, and
substitutes differential values calculated by differentiating the characteristic curve to said equation.

**Patentansprüche**

1. Vorrichtung für Berechnung von Funktion eines Kreislauforgans, die umfasst:

   eine Verengungs-Einheit (10), die so ausgeführt ist, dass sie einen Messobjekt-Teil eines Messobjektes verengt;
   eine Druckerfassungs-Einheit (30), die durch die Verengungs-Einheit erzeugten Verengungs-Druck erfasst;
   eine Drucksteuerungs-Einheit (20), die den Verengungs-Druck ändert;
   eine Einheit (40) für Erfassung von Pulswellen, die Pulswellen auf Basis eines Ausgangssignals von der Druckerfassungs-Einheit während eines durch die Drucksteuerungs-Einheit durchgeführten Prozesses zum Ändern des Verengungs-Drucks erfasst; und
   **gekennzeichnet durch**:
   eine Einheit (50) für Berechnung von Kenngrößen, die:

   kumulativ addierte Pulswellen-Werte durch kumulatives Addieren einer Fläche der Pulswellen erzeugt, die jeweils den Verengungs-Drücken entsprechen,
   kumulative Additionsverhältnisse durch Normalisieren der kumulativ addierten Pulswellen-Werte berechnet, und die kumulativen Additionsverhältnisse in eine Gleichung einsetzt, die Variablen und Koeffizienten enthält, die im Voraus über mehrdimensionale Analyse ermittelt werden.

2. Vorrichtung für Berechnung von Funktion eines Kreislauforgans nach Anspruch 1, wobei die Einheit (50) für Berechnung von Kenngrößen so ausgeführt ist, dass sie verwendet:

   einen geänderten Betrag der kumulativen Additionsverhältnisse, wenn der Verengungs-Druck um einen vorgegebenen Betrag geändert wird,
   eine Steigung einer Kennlinie, die das Verhältnis der kumulativen Additionsverhältnisse und des Verengungs-Drucks anzeigt, wenn der Verengungs-Druck um einen vorgegebenen Betrag geändert wird,
   einen Integralwert der Kennlinie der kumulativen Additionsverhältnisse, wenn der Verengungs-Druck um einen vorgegebenen Betrag geändert wird.

3. Vorrichtung für Berechnung von Funktion eines Kreislauforgans nach Anspruch 1, wobei die Einheit (50) für Berechnung von Kenngrößen so ausgeführt ist, dass sie verwendet:

   einen geänderten Betrag des Verengungs-Drucks, wenn die kumulativen Additionsverhältnisse um einen vorgegebenen Betrag geändert werden,
   eine Steigung einer Kennlinie, die die Beziehung zwischen dem Verengungs-Druck und den kumulativen Additionsverhältnissen anzeigt, wenn die kumulativen Additionsverhältnisse um einen vorgegebenen Betrag geändert werden,
   einen Integralwert der Kennlinie des Verengungs-Drucks, wenn die kumulativen Additionsverhältnisse um einen vorgegebenen Betrag geändert werden.

4. Vorrichtung für Berechnung von Funktion eines Kreislauforgans nach einem der Ansprüche 1 bis 3, wobei

   die Kenngröße der Funktion des Kreislauforgans ein Wert ist, der einen Gefäß-Zustand quantitativ angibt, und die Einheit (50) für Berechnung von Kenngrößen den Gefäß-Zustand anhand eines Berechnungsergebnisses der Kenngröße bewertet.

5. Vorrichtung zur Berechnung der Funktion eines Kreislauforgans nach einem der Ansprüche 1 bis 3, wobei die Einheit (50) zur Berechnung von Kenngrößen die Kenngröße mit einem Diskriminanz-Wert vergleicht, der zum Erkennen einer bestimmten KreislauforganErkrankung verwendet wird, und ein Erkennungsergebnis erzeugt, das einen Zustand des Messobjektes angibt, der mit der bestimmten Kreislauforganerkrankung zusammenhängt.

6. Vorrichtung für Berechnung von Funktion eines Kreislauforgans nach einem der Ansprüche 1 bis 4, wobei die mehrdimensionale Analyse eine Hauptkomponenten-Analyse ist.

**7.** Vorrichtung für Berechnung von Funktion eines Kreislauforgans nach einem der Ansprüche 1 bis 6, wobei die Einheit (50) für Berechnung von Kenngrößen:

eine Hüllkurve festlegt, die die Beziehung zwischen dem Verengungs-Druck und den kumulativen Additionsverhältnissen anzeigt, die Hüllkurve bei vorgegebenen geänderten Beträgen der Verengungs-Drücke oder vorgegebenen geänderten Beträgen der kumulativen Additionsverhältnisse unterteilt,
und Werte, die jeweils den unterteilten Abschnitten der Hüllkurve entsprechen, als die kumulativen Additionsverhältnisse verwendet.

**8.** Vorrichtung für Berechnung von Funktion eines Kreislauforgans nach Anspruch 1, wobei

die Einheit (50) für Berechnung von Kenngrößen eine Kennlinie festlegt, die die Beziehung zwischen der Fläche der Pulswellen und Verengungs-Drücken anzeigt, wenn die Pulswellen erfasst werden, und
die durch Differenzieren der Kennlinie berechneten Differenzwerte in die Gleichung einsetzt.

## Revendications

**1.** Dispositif de calcul du fonctionnement d'un organe circulatoire comprenant :

une unité de constriction (10) conçue pour contraindre une partie du sujet de mesure d'un sujet de mesure ;
une unité de détection de pression (30) qui détecte la pression de constriction générée par l'unité de constriction ;
une unité de régulation de pression (20) qui fait varier la pression de constriction ;
une unité de détection d'onde d'impulsion (40) qui détecte les ondes d'impulsion sur la base d'un signal de sortie depuis l'unité de détection de pression durant un procédé de variation de la pression de constriction effectué par l'unité de régulation de pression ; et
**caractérisé par**
une unité de calcul de quantité de caractéristique (50) qui :

génère des valeurs d'onde d'impulsion ajoutées de manière cumulée en ajoutant de manière cumulée une surface des ondes d'impulsion qui correspondent respectivement aux pressions de constriction,
calcule les ratios d'addition cumulés en normalisant les valeurs d'onde d'impulsion ajoutées de manière cumulée, et substitue les ratios d'addition cumulés en une équation comprenant des variables et des coefficients qui sont obtenus à l'avance à travers une analyse multivariable.

**2.** Dispositif de calcul d'organe circulatoire selon la revendication 1, l'unité de calcul de quantité de caractéristique (50) étant conçue pour utiliser l'un

d'une quantité variée des ratios d'addition cumulés lorsque la pression de constriction varie d'une quantité prédéterminée,
d'un gradient d'une courbe de caractéristique montrant la relation des ratios d'addition cumulés et la pression de constriction lorsque la pression de constriction varie d'une quantité prédéterminée,
d'une valeur intégrale de la courbe de caractéristique des ratios d'addition cumulés lorsque la pression de constriction varie d'une quantité prédéterminée.

**3.** Dispositif de calcul d'organe circulatoire selon la revendication 1, l'unité de calcul de quantité de caractéristique (50) étant conçue pour utiliser l'un

d'une quantité variée de la pression de constriction lorsque les ratios d'addition cumulés varient d'une quantité prédéterminée,
d'un gradient d'une courbe de caractéristique montrant la relation de la pression de constriction et des ratios d'addition cumulés lorsque les ratios d'addition cumulés varient d'une quantité prédéterminée,
d'une valeur intégrale de la courbe de caractéristique de la pression de constriction lorsque les ratios d'addition cumulés varient d'une quantité prédéterminée.

**4.** Dispositif de calcul du fonctionnement d'un organe circulatoire selon l'une quelconque des revendications 1 à 3,

la quantité de caractéristique du fonctionnement d'un organe circulatoire étant une valeur indiquant quantitativement un état vasculaire, et

l'unité de calcul de quantité de caractéristique (50) évaluant l'état vasculaire en utilisant un résultat de calcul de la quantité de caractéristique.

5. Dispositif de calcul du fonctionnement d'un organe circulatoire selon l'une quelconque des revendications 1 à 3, l'unité de calcul de quantité de caractéristique (50) comparant la quantité de caractéristique à une valeur discriminante utilisée pour distinguer une maladie d'organe circulatoire spécifique et générer un résultat de distinction indiquant un état du sujet de mesure lié à la maladie d'un organe circulatoire spécifique.

6. Dispositif de calcul du fonctionnement d'un organe circulatoire selon l'une quelconque des revendications 1 à 4, l'analyse multivariable étant une analyse en composantes principales.

7. Dispositif de calcul du fonctionnement d'un organe circulatoire selon l'une des revendications 1 à 6, l'unité de calcul de quantité de caractéristique (50) spécifiant une enveloppe montrant la relation de la pression de constriction et des ratios d'addition cumulés, divisant l'enveloppe à des quantités variées prédéterminées des pressions de constriction ou des quantités variées prédéterminées des ratios d'addition cumulés, et utilisant des valeurs correspondant respectivement aux portions divisées de l'enveloppe comme ratios d'addition cumulés.

8. Dispositif de calcul du fonctionnement d'un organe circulatoire selon la revendication 1,
l'unité de calcul de quantité de caractéristique (50) spécifiant une courbe de caractéristique montrant la relation de la surface des ondes d'impulsion et des pressions de constriction lorsque les ondes d'impulsion sont détectées, et substituant des valeurs différentielles calculées par différenciation de la courbe de caractéristique de ladite équation.

Fig.1

# Fig.2

Constriction
Pressure P

# Fig.3

Amplitude

# Fig.4A

Pulse Wave Area A

A1  A2  A3

C1  C2  C3

Cuff Pressure C

# Fig.4B

Cumulatively Added Pulse Wave Value B

B3 — A1+A2+A3

B2 — A1+A2

B1 — A1

C1  C2  C3

Cuff Pressure C

# Fig.5

(%)

100

Cumulative Addition Ratio D

L2

Low Pressure   C1 C2        High Pressure        Cuff Pressure C

## Fig.6

## Fig.7

Message: Your blood hardness is 8.5.
Blood vessel hardness level is 2. This is border range.

# Fig.8

# Fig.9

Message: You may be suffering from diabetes.

# Fig.10

# Fig.11

# Fig.12

# Fig.13

**EP 2 976 999 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2465422 A1
- JP 2005323853 A